# EUROPEAN PATENT APPLICATION

(11) **EP 4 826 733 A1**
(43) Date of publication of application: **21.10.2026**
(21) Application number: 24910389.6
(22) Date of filing: 15.11.2024
(51) Int. Cl.: G16H 30/40

(54) **SCANNING PROCESSING METHOD AND APPARATUS, DEVICE, AND MEDIUM**

(30) Priority: 25.12.2023 CN 202311798702
(71) Applicant: Shining 3D Tech Co., Ltd., Hangzhou, Zhejiang 311258 (CN)
(72) Inventor: XIAO, Qilin, Hangzhou, Zhejiang 311258 (CN); ZHAO, Xiaobo, Hangzhou, Zhejiang 311258 (CN); ZHANG, Huiquan, Hangzhou, Zhejiang 311258 (CN); NI, Kaijia, Hangzhou, Zhejiang 311258 (CN); MA, Chao, Hangzhou, Zhejiang 311258 (CN)
(74) Representative: Dehns Germany Partnerschaft mbB
(86) International application number: PCT/CN2024/132450
(87) International publication number: WO 2025/139454

(57) **Abstract**

The embodiments of the present invention relate to a method and an apparatus for scanning processing, a device and a medium. The method comprises: in response to an occlusal relationship addition request, acquiring a target dental arch and an initial occlusal relationship corresponding to the target dental arch; scanning the target dental arch in a target occlusal state to obtain an occlusal piece model; and performing calculation based on the initial occlusal relationship and the occlusal piece model to obtain a target occlusal relationship and storing same.

## Description

The present application claims priority to Chinese Patent Application no. 202311798702.4, to the China National Intellectual Property Administration on December 25, 2023 and entitled "Method and Apparatus for scanning processing, Device and Medium", which is incorporated herein by reference in its entirety.

### Technical Field

The present invention relates to the field of scanning processing technologies, and in particular, to a method and an apparatus for scanning processing, a device and a medium.

### Background

Currently, in dental diagnosis and treatment, occlusal analysis is an important step in evaluating the patient's occlusal relationship. By analyzing the contact conditions and occlusal balance of the patient's teeth, dentists can understand the functional status of the patient's oral cavity, identify potential occlusal problems, and formulate corresponding treatment plans. For example, when performing dental restorations (such as crown restorations, bridge restorations, or dentures), dentists need to ensure that the restoration has a good occlusal relationship with the patient's other teeth. Inaccurate occlusal relationships may lead to poor fit of the restoration, functional disorders, and other issues.

In the related art, existing intra-oral scanning software can only acquire a single set of occlusal position relationship, that is, the occlusal position relationship when the patient's maxilla and mandible are in a natural occlusal state. This limitation makes it impossible to comprehensively understand the tooth contact conditions at different positions in the patient's oral cavity, which may lead to inaccurate evaluation of the occlusal relationship. Furthermore, during dental restoration procedures, dentists need to ensure that the restoration maintains a good occlusal relationship with the patient's other teeth. Relying on only one set of natural occlusal relationship data makes it difficult to fully guarantee the fit and functionality of the restoration.

### Summary

In order to solve the described technical problem or at least partially solve the described technical problem, the present invention provides a method and an apparatus for scanning processing, a device and a medium.

The embodiments of the present invention further provide a method for scanning processing, including:
in response to an occlusal relationship addition request, acquiring a target dental arch and an initial occlusal relationship corresponding to the target dental arch;
scanning the target dental arch to obtain an occlusal piece model, wherein the target dental arch is in a target occlusal state;
performing calculation based on the initial occlusal relationship and the occlusal piece model to obtain a target occlusal relationship and storing the target occlusal relationship.

The embodiments of the present invention further provide an apparatus for scanning processing, including:
a first response acquisition module, configured to acquire, in response to an occlusal relationship addition request, a target dental arch and an initial occlusal relationship corresponding to the target dental arch;
a scanning module, configured to scan the target dental arch to obtain an occlusal piece model, wherein the target dental arch is in a target occlusal state;
a calculation and storage module, configured to perform calculation based on the initial occlusal relationship and the occlusal piece model to obtain a target occlusal relationship and store the target occlusal relationship.

The embodiments of the present invention further provide an electronic device. The electronic device includes: a processor; a memory configured to store an executable instruction executable by the processor; wherein the processor configured to read the executable instruction from the memory and execute the instruction to implement the method for scanning processing provided in the embodiments of the present invention.

The embodiments of the present invention further provide a computer readable storage medium, which stores a computer program, wherein the computer program is configured to execute the method for scanning processing provided in the embodiments of the present invention.

Compared with the prior art, the technical solutions provided in the embodiments of the present invention have the following advantages: in the scanning processing scheme provided in the embodiments of the present invention, a target dental arch and an initial occlusal relationship corresponding to the target dental arch are acquired in response to an occlusal relationship addition request, the target dental arch is in a target occlusal state; the target dental arch is scanned to obtain an occlusal piece model; and calculation is performed based on the initial occlusal relationship and the occlusal piece model to obtain and store a target occlusal relationship. By means of the technical solution, as required, a user can automatically add multiple groups of occlusal positions for scanning, so that data of the multiple occlusal positions can be acquired, and more comprehensive and accurate occlusal analysis can be provided, thereby better learning about the occlusion condition of a dental arch on the basis of the multiple groups of occlusal relationships, so as to improve the processing quality of subsequent dental restorations, dental implant, etc.

### Brief Description of the Drawings

The above and other features, advantages, and aspects of various embodiments of the present invention will become more apparent from the following detailed description taken in conjunction with the accompanying drawings. Throughout the drawings, the same or similar reference numbers refer to the same or similar elements. It should be understood that the drawings are schematic and that originals and elements are not necessarily drawn to scale.
Fig. 1 is a schematic flowchart of a method for scanning processing according to an embodiment of the present invention;
Fig. 2 is a schematic flowchart of another method for scanning processing according to an embodiment of the present invention;
Fig. 3 is a schematic diagram of information display according to an embodiment of the present invention;
Fig. 4 is a schematic diagram of an occlusal relationship according to an embodiment of the present invention;
Fig. 5 is a schematic flowchart of a scanning procedure according to an embodiment of the present invention;
Fig. 6 is a schematic diagram of post-processing after scanning according to an embodiment of the present invention;
Fig. 7a is a schematic flowchart of yet another method for scanning processing according to an embodiment of the present invention;
Fig. 7b is a schematic diagram of another information display according to an embodiment of the present invention;
Fig. 8 is a schematic flowchart of still another method for scanning processing according to an embodiment of the present invention;
Fig. 9 is a schematic flowchart of a further method for scanning processing according to an embodiment of the present invention;
Fig. 10 is a schematic structural diagram of a scanning processing apparatus according to an embodiment of the present invention;
Fig. 11 is a schematic structural diagram of an electronic device according to an embodiment of the present invention.

### Detailed Description of the Embodiments

Embodiments of the present invention are described in more detail below with reference to the drawings. Although certain embodiments of the present invention are shown in the drawings, it should be understood that the present invention may be implemented in various forms and should not be construed as being limited to the embodiments described here. On the contrary, these embodiments are provided to understand the present invention more clearly and completely. It should be understood that the drawings and the embodiments of the present invention are only for exemplary purposes and are not intended to limit the scope of protection of the present invention.

It should be understood that various steps recorded in the implementation modes of the method of the present invention may be performed according to different orders and/or performed in parallel. In addition, the implementation modes of the method may include additional steps and/or steps omitted or unshown. The scope of the present invention is not limited in this aspect.

The term "including" and variations thereof used in this article are open-ended inclusion, i.e. "including but not limited to". The term "based on" refers to "at least partially based on". The term "one embodiment" means "at least one embodiment"; the term "another embodiment" means "at least one other embodiment"; and the term "some embodiments" means "at least some embodiments". Relevant definitions of other terms may be given in the description hereinafter.

It should be noted that concepts such as "first" and "second" mentioned in the present invention are only used to distinguish different apparatuses, modules or units, and are not intended to limit orders or interdependence relationships of functions performed by these apparatuses, modules or units.

It should be noted that, modifications of "one" and "more" mentioned in the present invention are schematic rather than restrictive, and those skilled in the art should understand that unless otherwise explicitly stated in the context, it should be understood as "one or more".

The names of messages or information exchanged between multiple apparatuses in embodiments of the present invention are for illustrative purposes only, and are not intended to limit the scope of these messages or information.

Specifically, if intra-oral scanning software only supports the acquisition of a single set of occlusal relationships, that is, after the patient occludes the maxilla and mandible in a specific manner, the software scans the left occlusal position, right occlusal position, and anterior occlusal position. Each occlusion changes the relative spatial position of the maxilla and mandible by means of stitching, continuously refining the occlusal relationship. Ultimately, it outputs a single set of maxilla and mandible models with relative positional relationships. If the doctor wants to obtain more maxillomandibular relative position relationship, the doctor needs to scan the maxilla and the mandible again and the occlusion. Scanning the same maxilla and mandible again not only wastes time for dentists and patients, but also the outputted maxilla model and mandible models are not in the same spatial coordinate system as the maxilla model and mandible models scanned previously, which causes a certain obstacle to the doctor when comparing a plurality of occlusal relationships.

Therefore, scanning software is required to provide a manner capable of acquiring multiple groups of occlusal positions, thereby offering dentists comprehensive and accurate occlusal analysis data, assisting them in making more precise diagnoses and treatment decisions, as well as evaluating the contact between a restoration and the patient's teeth, so as to adjust the shape and size of the restoration for better fit and functionality.

The embodiments of the present invention provide a method for scanning processing, which solves the problem in the prior art that evaluation can only be performed at specific occlusal positions, making it impossible to fully understand the tooth contact conditions at different positions in the patient's oral cavity, which may lead to inaccurate evaluation of the occlusal relationship, thereby affecting the quality of tooth restoration. The method allows scanning of multiple sets of occlusal relationships according to the dentist's needs, provides more comprehensive and accurate occlusal position relationships, and helps dentists better understand the patient's occlusal condition. For example, during the tooth restoration process, data of multiple occlusal positions can be provided to help the dentist evaluate the contact between the restoration and the patient's teeth at different occlusal positions, thereby adjusting the shape and size of the restoration to achieve a better fit. For another example, in conjunction with occlusal space measurement, it assists the dentist in confirming the occlusal clearance under multiple occlusal states, and for full-arch implantation, utilizing these multiple occlusal positions to assist in confirming the occlusal relationship during the subsequent design stage.

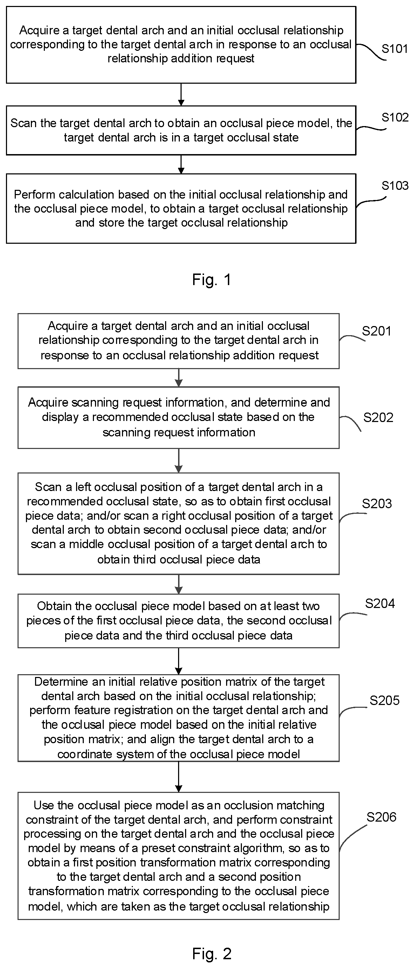

Specifically, Fig. 1 is a schematic flowchart of a method for scanning processing according to an embodiment of the present invention. The method may be executed by a scanning processing apparatus. The apparatus may be implemented by software and/or hardware, and may generally be integrated in an electronic device. As shown in Fig. 1, the method includes:
step 101: a target dental arch and an initial occlusal relationship corresponding to the target dental arch are acquired in response to an occlusal relationship addition request.

The target dental arch may be the user's actual dental arch or a dental model such as a plaster cast. Depending on the application scenario, the target dental arch can be scanned using a hand-held intra-oral scanner, an extra-oral scanner, or a desktop scanner.

In some embodiments, the scanning procedure may include scanning of the maxilla, the mandible and the occlusion, and the occlusion may be understood as acquiring an occlusal position relationship between the maxilla and the mandible by scanning. The occlusion scan usually includes a set of scanning for the natural occlusal relationship, named "maximum intercuspation position" , which cannot be renamed or deleted.

In the embodiment of the present invention, the window of an occlusal relationship list may be opened by clicking a "multiple occlusal relationships" control button of an occlusion scanning interface. In this window, other occlusal relationships can be switched, added, deleted, or renamed. Specifically, the occlusal relationship addition request is triggered by clicking an "add" control button.

In the embodiments of the present invention, before responding to the occlusal relationship addition request, at least one set of occlusal relationships already exists. For example, when there exists only the "maximum intercuspation position" occlusal relationship, the "maximum intercuspation position" can be used as the initial occlusal relationship; for another example, when there exists only an occlusal relationship 1, the occlusal relationship 1 can be used as the initial occlusal relationship; for yet another example, when there exist both the "maximum intercuspation position" and the occlusal relationship 1, the "maximum intercuspation position" needs to be used as the initial occlusal relationship."

In the embodiment of the present invention, after responding to an occlusal relationship addition request, a target dental arch and an initial occlusal relationship corresponding to the target dental arch may be acquired, i.e. the maxillomandibular relative position relationship in the target dental arch.

Step 102: the target dental arch is scanned to obtain an occlusal piece model, the target dental arch is in a target occlusal state;
wherein the target occlusal state can be selected and determined as needed. For example, the target occlusal state can be determined by manually controlling the target dental arch to be at a specific occlusal position by means of user operation. Alternatively, analysis can be performed on the basis of inputted scanning request information to generate a recommended occlusal state and display same to the user, so that the dentist can prompt the user to adjust the dental occlusal state, thereby placing the user's corresponding target dental arch in the recommended occlusal state as the target occlusal state, further meeting user needs and improving the scanning efficiency and flexibility.

Specifically, for scanning each set of occlusal relationships, while the user maintains the same occlusal position, all or some of the left, right, and anterior sides can be scanned respectively, thereby obtaining two or three pieces of occlusion data.

In the embodiments of the present invention, there are many ways to scan the target dental arch to obtain an occlusal piece model. In some implementations, first occlusal piece data is obtained by scanning the left occlusal position of the target dental arch, and second occlusal piece data is obtained by scanning the right occlusal position of the target dental arch, and the occlusal piece model is obtained on the basis of the first occlusal piece data and the second occlusal piece data.

In other implementations, first occlusal piece data is obtained by scanning the left occlusal position of the target dental arch, second occlusal piece data is obtained by scanning the right occlusal position of the target dental arch, and third occlusal piece data is obtained by scanning the middle occlusal position of the target dental arch, and the occlusal piece model is obtained on the basis of the first occlusal piece data, the second occlusal piece data, and the third occlusal piece data.

The above are merely examples, scanning may be performed at two sides, three sides, or four or more sides as needed, which is not specifically limited in the embodiments of the present invention.

Step 103: calculation is performed based on the initial occlusal relationship and the occlusal piece model, to obtain a target occlusal relationship and store the target occlusal relationship.

In the embodiments of the present invention, the initial occlusal relationship refers to a relative position relationship between a maxilla and a mandible in a target dental arch. After an occlusal piece model is acquired, calculation is performed based on the initial occlusal relationship and the occlusal piece model, to obtain a target occlusal relationship and store the target occlusal relationship.

Specifically, for scanning each side of the occlusal piece, a maxillomandibular relative position can be obtained by means of feature registration and multi-constraint processing. Feature registration can be understood as splicing the maxilla and mandible models with the current piece of occlusion data being scanned, according to a series of features such as geometric shape, surface texture, normal direction, and color information, thereby aligning the maxilla and the mandible to the coordinate system of the current piece of occlusion data.

In some embodiments, an initial relative position matrix of the target dental arch is determined based on the initial occlusal relationship; feature registration is performed on the target dental arch and the occlusal piece model based on the initial relative position matrix, and the target dental arch is aligned to a coordinate system of the occlusal piece model; the occlusal piece model is used as an occlusion matching constraint of the target dental arch; and constraint processing is performed on the target dental arch and the occlusal piece model by means of a preset constraint algorithm, so as to obtain a first position transformation matrix corresponding to the target dental arch and a second position transformation matrix corresponding to the occlusal piece model, which are taken as the target occlusal relationship.

In some embodiments, a current position transformation matrix of the current piece of occlusion data and a previous position transformation matrix of a previous piece of occlusion data are acquired; constraint processing is performed on a target dental arch to obtain a target position transformation matrix; calculation is performed based on the current position transformation matrix, the previous position transformation matrix and the target position transformation matrix to obtain an updated position transformation matrix of the previous piece of occlusion data; and the first position transformation matrix corresponding to the target dental arch and the updated position transformation matrix of the previous piece of occlusion data are used as a target occlusal relationship.

In the embodiments of the present invention, there may be one or more target occlusal relationships; and storing the target occlusal relationship may be understood as storing the occlusion name of the occlusal position, and the relative position matrix of the maxilla model and the mandible model with respect to the initial occlusal relationship.

In the scanning processing scheme provided in the embodiments of the present invention, a target dental arch and an initial occlusal relationship corresponding to the target dental arch are acquired in response to an occlusal relationship addition request, the target dental arch being in a target occlusal state; the target dental arch is scanned to obtain an occlusal piece model; and calculation is performed based on the initial occlusal relationship and the occlusal piece model to obtain and store a target occlusal relationship. By means of the technical solution, as required, a user can automatically add multiple groups of occlusal positions for scanning, so that data of the multiple occlusal positions can be acquired, and more comprehensive and accurate occlusal analysis can be provided, thereby better learning about the occlusion condition of a dental arch on the basis of the multiple groups of occlusal relationships, so as to improve the processing quality of subsequent dental restorations, dental implant, etc.

Fig. 2 is a schematic flowchart of another method for scanning processing according to an embodiment of the present invention. In this embodiment, based on the foregoing embodiment, the foregoing method for scanning processing is optimized. As shown in Fig. 2, the method includes:
step 201: a target dental arch and an initial occlusal relationship corresponding to the target dental arch are acquired in response to an occlusal relationship addition request.

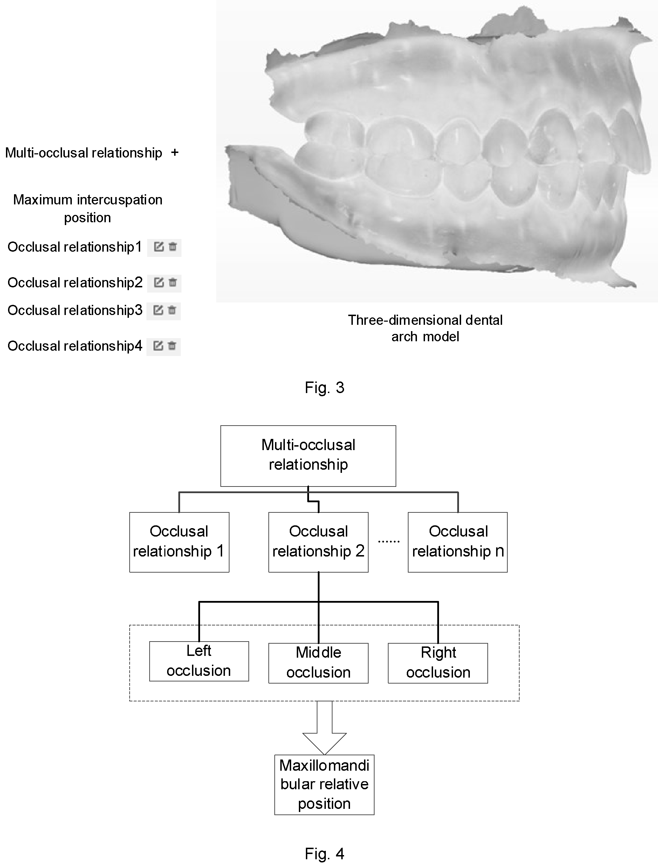

Exemplarily, Fig. 3 is a schematic diagram of information display according to an embodiment of the present invention. The figure shows a schematic diagram of an occlusion scanning interface, which includes a target dental arch in an initial occlusal relationship, and the "multiple occlusal relationships" button on the occlusion scanning interface is clicked to open an occlusal relationship list window. As shown in the figure, in this window, other occlusal relationships can be switched, added, deleted, or renamed. For example, by clicking an "add" button control of the occlusal relationship 1, an occlusal relationship addition request may be triggered, so that a target dental arch and an initial occlusal relationship corresponding to the target dental arch may be acquired in response to the occlusal relationship addition request.

Acquiring the initial occlusal relationship corresponding to the target dental arch may be understood as acquiring the relative position matrix corresponding to the initial occlusal relationship of the target dental arch.

Step 202: scanning request information is acquired, and a recommended occlusal state based on the scanning request information is determined and displayed.

In the embodiments of the present invention, different scanning orders in the scanning request information, such as tooth restoration and tooth implantation, have different requirements for occlusal positions. Therefore, a recommended occlusal state can be determined and displayed based on the scanning orders in the scan request information, so that the dentist can prompt the user to adjust the dental occlusal state, thereby placing the user's corresponding target dental arch in the recommended occlusal state, and the subsequently acquired target occlusal relationship will better meet the requirements.

Step 203: a left occlusal position of a target dental arch in a recommended occlusal state is scanned, so as to obtain first occlusal piece data; and/or a right occlusal position of a target dental arch is scanned to obtain second occlusal piece data; and/or a middle occlusal position of a target dental arch is scanned to obtain third occlusal piece data.

Step 204: the occlusal piece model is obtained based on at least two pieces of the first occlusal piece data, the second occlusal piece data and the third occlusal piece data.

Specifically, for scanning each set of occlusal relationships, while the user maintains the same occlusal position, all or some of the left, right, and anterior sides can be scanned respectively, thereby obtaining three pieces of occlusion data. Since each piece of occlusion data contains both partial maxillary data and partial mandibular data, the multiple pieces of occlusal piece data obtained by scanning from multiple side positions form multiple constraints that can be used to determine the relative position of the maxilla and mandible, allowing for a more accurate determination of the current occlusal relationship between the maxilla and mandible on the basis of a multi-constraint matching algorithm.

It should be noted that, in order to improve the scanning processing efficiency, the occlusion data on the left side and the right side may be scanned; in addition, if teeth are missing on one side, two pieces of occlusion data on one side and the front side may be scanned. The occlusion scanning on each side obtains a piece of occlusion data.

Exemplarily, Fig. 4 is a schematic diagram of an occlusal relationship according to an embodiment of the present invention, and shows a schematic diagram of one occlusal relationship. The multiple occlusal relationships may include an occlusal relationship 1, an occlusal relationship 2 to an occlusal relationship n, and each occlusal relationship may be obtained by scanning one or more of left occlusion, middle occlusion and right occlusion, so as to obtain the maxillomandibular relative position.

Step 205: an initial relative position matrix of the target dental arch is determined based on the initial occlusal relationship; feature registration is performed on the target dental arch and the occlusal piece model based on the initial relative position matrix; and the target dental arch is aligned to a coordinate system of the occlusal piece model.

Step 206: the occlusal piece model is used as an occlusion matching constraint of the target dental arch, and constraint processing is performed on the target dental arch and the occlusal piece model by means of a preset constraint algorithm, so as to obtain a first position transformation matrix corresponding to the target dental arch and a second position transformation matrix corresponding to the occlusal piece model, which are taken as the target occlusal relationship.

Specifically, for scanning each side of the occlusal piece, a maxillomandibular relative position can be obtained by means of feature registration and multi-constraint processing (such as MeshICP (Iterative Closest Point)).

Feature registration can be understood as splicing the maxilla and mandible models with the current piece of occlusion data being scanned, according to a series of features such as geometric shape, surface texture, normal direction, and color information, thereby aligning the maxilla and the mandible to the coordinate system of the current piece of occlusion data. Multi-constraint Mesh ICP (i.e. the algorithm used in three-dimensional model splicing, which combines a plurality of constraint conditions to improve the registration precision and stability of point cloud data) needs to be implemented on the basis of feature registration, that is, data of a plurality of scanned occlusal pieces as an occlusion matching constraint for the maxilla and the mandible is used to perform refined constraint processing between a plurality of grids, to obtain a more accurate relative position relationship between the maxilla, the mandible and the occlusal pieces.

It can be understood that the maxillary and mandibular models and the current piece of occlusion data being scanned are aligned to the same coordinate system. A preset number of feature points are respectively selected from the maxillary and mandibular models and the current piece of occlusion data; a relative position matrix between the feature points in the maxillary and mandibular models and the feature points in the current piece of occlusion is determined; and the maxillary and mandibular models are then spliced with the current piece of occlusion based on the relative position matrix to obtain a splicing result. In addition, splicing adjustment is performed on the splicing result by means of an ICP (Iterative Closest Point, Iterative closest point) algorithm, that is, overlapping data in the splicing result is processed to obtain a rotation translation matrix, and the current piece of occlusion data is transformed into a coordinate system of the maxillary and mandibular models based on the rotation translation matrix, to determine errors between the transformed occlusion data and the maxillary and mandibular models, until the errors are less than or equal to a preset error threshold, so as to obtain a splicing result. The error threshold is selectively set according to an application scenario.

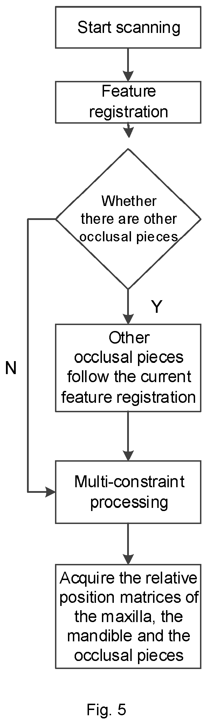

Exemplarily, Fig. 5 is a schematic flowchart of a scanning procedure according to an embodiment of the present invention, as shown in Fig. 5, a target dental arch is scanned; feature registration is performed on data of the target dental arch and the occlusal piece data; it is determined whether there is other occlusal piece data; multi-constraint processing is directly performed when there is no other occlusal piece data; when there is other occlusal piece data, feature registration is performed on the other occlusal piece data and the currently spliced features, and then multi-constraint processing is performed to obtain a relative position matrix of the maxilla, the mandible and each piece of occlusal piece data.

In some embodiments, the occlusal piece model includes at least two pieces of occlusion data; a current position transformation matrix of the current piece of occlusion data and a previous position transformation matrix of a previous piece of occlusion data are acquired; a target position transformation matrix of a target dental arch after constraint processing is acquired; calculation is performed based on the current position transformation matrix, the previous position transformation matrix and the target position transformation matrix to obtain an updated position transformation matrix of the previous piece of occlusion data.

Specifically, in a set of occlusion scans, if other occlusal pieces of the current set have already been scanned before scanning the current occlusal piece, after feature registration is completed, it is also necessary to adjust the relative position matrix of the previously scanned occlusal pieces to adapt to the spatial positions of the maxilla and mandible under the feature registration of the current occlusal piece. This ensures that two or three pieces of occlusion data can all be spatially matched with a unique maxillary and mandibular model, and that during multi-constraint processing, each piece of occlusion data, the maxilla and the mandible are all located at a relatively correct initial relative position.

For example, using the maxilla as a reference point, the relative position matrix of a previous occlusal piece data is always adjusted following the maxilla. That is, the occlusal piece is first restored to its original spatial position (ensuring the same spatial coordinate system) and then adjusted to the position of the current occlusal piece data. For example, the relative position matrix for splicing the maxilla to the current piece of occlusion data is uRt. After the last MeshICP processing of the maxilla, the relative position matrix of the maxilla is lastRt. The relative position matrix of a non-current occlusal piece data is tRt. The relative position matrix that needs to be changed for the non-current occlusal piece is tRt1, where tRt1=uRt**lastRt*⁻¹*tRt.

It should be noted that, when the multi-constraint Mesh ICP is performed, all scanned occlusal piece data also needs to be used as constraint conditions, and finally the relative position relationship between the maxilla, the mandible, and the current occlusal piece data is obtained.

In some embodiments, orientation correction is performed on the target dental arch to obtain an incremental relative position matrix; the first position transformation matrix is multiplied by the incremental relative position matrix to obtain a first target position transformation matrix corresponding to the target dental arch; the second position transformation matrix is multiplied by the incremental relative position matrix to obtain a second target position transformation matrix corresponding to the occlusal piece model; and the first target position transformation matrix and the second target position transformation matrix are then used as the target occlusal relationship.

Specifically, after multi-position occlusion scanning, post-processing optimization is performed on the mesh of each scanned set of occlusal relationships. Then, multi-constraint processing is performed again on the optimized mesh of each set of occlusal relationships, using the meshes of the maxilla, mandible, and all occlusal piece data in the current occlusal relationship set as constraint parameters to finely adjust the relative positions of the maxilla, mandible, and all occlusal piece data. Finally, orientation correction is performed, i.e., the teeth of the maxillary and mandibular models are aligned to face forward.

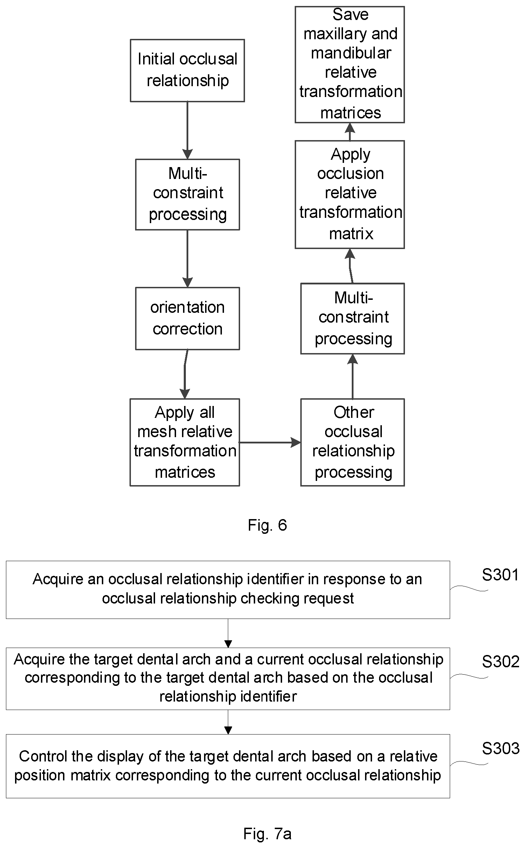

Exemplarily, Fig. 6 is a schematic diagram of post-processing after scanning according to an embodiment of the present invention. As shown in Fig. 6, first, the initial occlusal relationship (for example, maximum intercuspation) is processed by performing multi-constraint processing, followed by orientation correction of the mandible to obtain an incremental relative position matrix (incremental RT), which is the relative position matrix required to be adjusted on the basis of the current spatial position of the mandible. Then, the meshes of the maxilla, mandible, and the initial occlusal relationship are all multiplied by the incremental relative position matrix to obtain the final maxillomandibular relative position, as well as the relative position matrices of the occlusal piece data (the RT described in the figure). Finally, these relative position matrices are applied to their respective models, with the relative position matrix applied to the maxilla recorded as dURt, and the relative position matrix applied to the mandible recorded as dLRt.

Since there is only one set of maxillary and mandibular models, the spatial positions of the maxilla and mandible for other occlusal relationships are obtained by performing relative position matrix transformations on the basis of the maxilla and mandible of the initial occlusal relationship. Therefore, for other occlusal relationships, only multi-constraint processing is required, and orientation correction is not needed. However, the spatial positions of the maxilla, mandible, and the occlusal piece during multi-constraint processing must be the same as those at the time of scanning. Therefore, on the basis of the maxilla and mandible of the initial occlusal relationship, the relative position matrix of the maxilla recorded during scanning is denoted as uRt, the relative position matrix of the mandible is denoted as lRt, and the relative position matrix of each piece of occlusal piece data is denoted as tRt. For the input to the multi-constraint processing, the relative position matrix of the maxilla is uRt1, the relative position matrix of the mandible is lRt1, and the relative position matrix of the occlusal piece data is tRt1; and then uRt1=uRt* duRt^⁻¹; lRt1=lRt* dlRt^⁻¹; tRt1=tRt.

It should be noted that, since the occlusal piece does not depend on the default occlusal relationship, its input relative position matrix remains unchanged. Therefore, the relative position matrix of the occlusion can be directly applied to each occlusal piece.

The scanning processing solution provided in the embodiments of the present invention solves the problem in the prior art that evaluation can only be performed at specific occlusal positions, making it impossible to fully understand the tooth contact conditions at different positions in the patient's oral cavity, which may lead to inaccurate evaluation of the occlusal relationship, thereby affecting the quality of tooth restoration. The method allows scanning of multiple sets of occlusal relationships based on the dentist's needs, provides more comprehensive and accurate occlusal position relationships, and helps dentists better understand the patient's occlusal condition, so as to better perform dental restorations and dental implantation.

Fig. 7a is a schematic flowchart of yet another method for scanning processing according to an embodiment of the present invention.

In this embodiment, based on the foregoing embodiment, the foregoing method for scanning processing is optimized. As shown in Fig. 7a, the method includes:
step 301: an occlusal relationship identifier is acquired in response to an occlusal relationship checking request;
step 302: the target dental arch and a current occlusal relationship corresponding to the target dental arch are acquired based on the occlusal relationship identifier; and
step 303: the display of the target dental arch is controlled based on a relative position matrix corresponding to the current occlusal relationship.

It can be understood that after being added, one or more target occlusal relationships can be viewed. In the embodiments of the present invention, during the scanning phase, relevant data needs to be stored, such as the temporary mesh models of the maxilla and mandible obtained during scanning, the occlusion identifier (e.g., occlusion name) for each set of occlusal positions, the temporary mesh model of each piece of occlusal piece data, and the relative transformation matrices after mutual matching and alignment of the maxilla, the mandible, and the occlusal piece data. During scanning, these pieces of multi-position occlusion related information are stored and managed by means of corresponding model data files and project files, so that they can be reloaded and used in the post-processing phase when needed.

It can also be understood that after the post-processing is completed, the relevant data stored during the scanning stage will be deleted. The final data that are stored and managed include: the position corresponding to the initial occlusal relationship; the final post-processed mesh models of the maxilla and mandible after orientation correction, which means that the maxillary and mandibular relative position matrix corresponding to the initial occlusal relationship and the incremental relative position matrix for the orientation correction have already been applied to the maxillary and mandibular models, respectively; for each of the other sets of occlusion positions (excluding the initial occlusal relationship): the occlusion identifier and the relative position matrix of the maxillary and mandibular models with respect to the initial occlusion position (the maxillary and mandibular data are stored only once, while only the relative position matrices are recorded for the other occlusion positions); for each set of occlusion positions: the final post-processed mesh models of each occlusal piece data at the corresponding occlusion position, which means that each occlusal piece has already had the relative position matrix determined by the corresponding occlusal relationship applied to it.

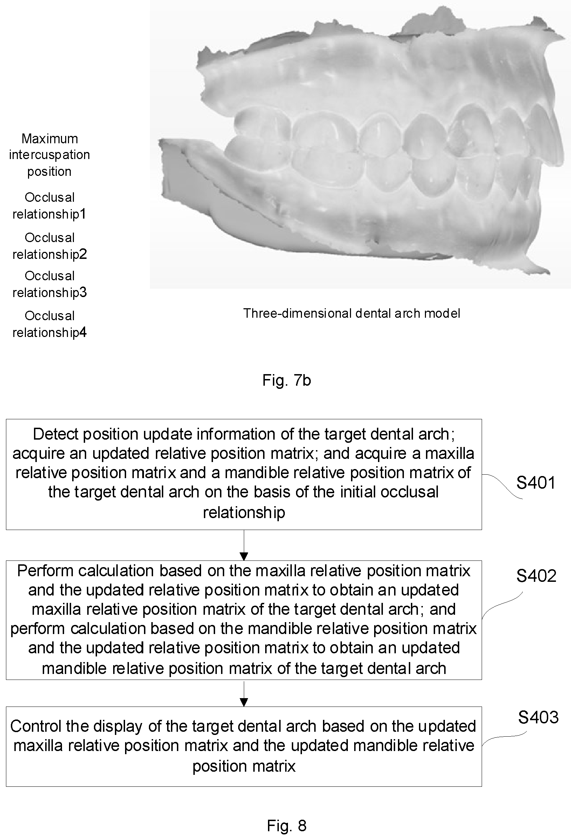

Exemplarily, by clicking the occlusal relationship list window shown in Fig. 7b, such as clicking "occlusal relationship 1", an occlusal relationship view request is triggered; the occlusal relationship identifier "occlusal relationship 1" is acquired; and on the basis of the "occlusal relationship 1", the target dental arch and the corresponding relative position matrix Z1 corresponding thereto are acquired; and the display of the target dental arch is then controlled based on the relative position matrix Z1.

Specifically, a list of multi-occlusal relationship names is obtained on the basis of the occlusal relationship information. By clicking, a user can switch between occlusal relationships to view the relative spatial relationship between the maxilla, mandible, and occlusal pieces. Each time an occlusal relationship is switched, the maxillomandibular relative position matrix information corresponding to the current occlusal relationship is obtained from a specified file, the relative position matrix of the maxillary and mandibular models is set, and the occlusal position relationship is displayed.

In some embodiments, as shown in Fig. 8, the method further includes:
step 401: position update information of the target dental arch is detected; an updated relative position matrix is acquired; and a maxilla relative position matrix and a mandible relative position matrix of the target dental arch are acquired on the basis of the initial occlusal relationship;
step 402: calculation is performed based on the maxilla relative position matrix and the updated relative position matrix to obtain an updated maxilla relative position matrix of the target dental arch; and calculation is performed based on the mandible relative position matrix and the updated relative position matrix to obtain an updated mandible relative position matrix of the target dental arch; and
step 403: the display of the target dental arch is controlled based on the updated maxilla relative position matrix and the updated mandible relative position matrix.

Specifically, both the maxillary and mandibular models have a default spatial position. When this default position changes, for example, after coordinate adjustment, the maxillomandibular relative position matrixes for the occlusal relationships recorded in the storage file need to be changed accordingly.

Specifically, let the value of the adjusted relative position matrix be denoted as rt. In the occlusal relationship of the storage file, the relative position matrix of the maxilla is recorded as uRt, the relative position matrix of the mandible is recorded as lRt, the RT of the adjusted maxilla is recorded as uRt1, and the RT of the adjusted mandible is recorded as lRt1, then uRt1=rt*uRt*rt^⁻¹; lRt1=rt*lRt*rt^⁻¹.

In some embodiments, each occlusal relationship and an opposing dental arch corresponding to each occlusal relationship are outputted in response to an occlusal relationship output request,
wherein when the opposing dental arch is a mandible, the relative position matrix of the opposing dental arch corresponding to each of the occlusal relationships is a relative position inverse matrix of the maxilla multiplied by a relative position of the mandible, and the relative position matrix of the occlusal piece corresponding to each of the occlusal relationships is a relative position inverse matrix of the maxilla;
when the opposing dental arch is a maxilla, the relative position matrix of the opposing dental arch corresponding to each of the occlusal relationships is a relative position inverse matrix of a mandible multiplied by a relative position of a maxilla, and the relative position matrix of the occlusal piece corresponding to each of the occlusal relationships is a relative position inverse matrix of the mandible.

Specifically, when exporting mesh files for multiple occlusal relationships, the mesh of the opposing dental arch (non-working arch; when creating the scan order, either the maxilla or the mandible can be determined as the working arch, and conversely, the non-working arch is the opposing dental arch; if both the maxilla and mandible are working arches or non-working arches, the mandible is set as the opposing dental arch by default) corresponding to each occlusal relationship is additionally outputted. The relative position matrix of the opposing dental arch is obtained by calculating the relative position matrix of the maxilla and mandible recorded in the occlusal relationship, and finally the opposing dental arch is stored as a mesh file with that relative position matrix.

Specifically, if the mandible is the opposing dental arch, let the RT of the maxilla recorded in the occlusal relationship be denoted as uRt, the RT of the mandible as lRt, the RT of the opposing dental arch in the exported mesh file is Irtl, and the RT of the occlusal piece is tRtl, then lRt1=uRt^⁻¹*lRt; tRt1= uRt^⁻¹.

If the maxilla is the opposing dental arch, let the RT of the maxilla recorded in the occlusal relationship be denoted as uRt, the RT of the mandible as lRt, the RT of the opposing dental arch in the exported mesh file is uRtl, and the RT of the occlusal piece is tRtl, then . uRt1=lRt^⁻¹*uRt; tRt1= lRt^⁻¹.

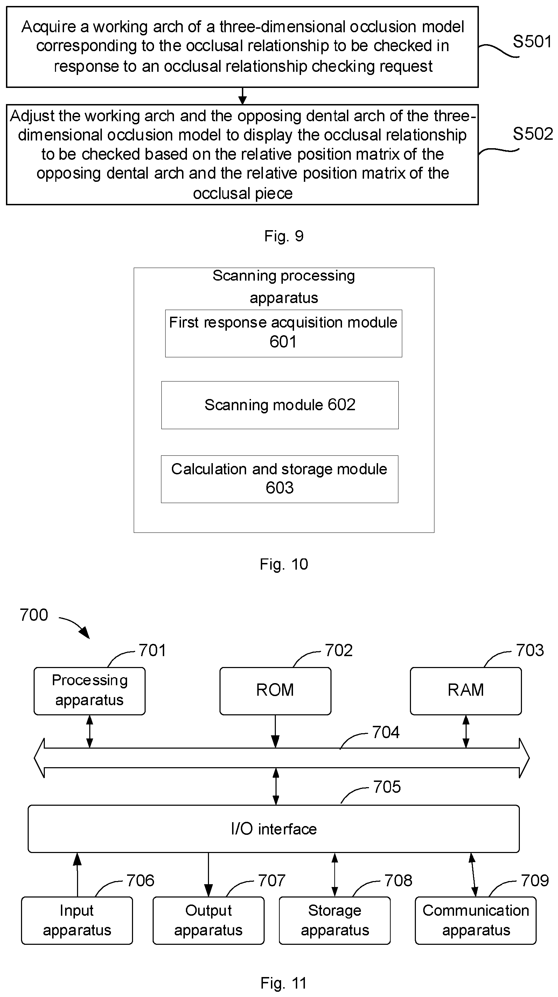

In some embodiments, as shown in Fig. 9, the method further includes:
step 501: a working arch of a three-dimensional occlusion model corresponding to the occlusal relationship to be checked is acquired in response to an occlusal relationship checking request; and
step 502: the working arch and the opposing dental arch of the three-dimensional occlusion model to display the occlusal relationship to be checked are adjusted based on the relative position matrix of the opposing dental arch and the relative position matrix of the occlusal piece.

Thus, when the exported file is loaded into other display software, on the basis of an occlusal relationship viewing request, a working arch of a three-dimensional occlusion model corresponding to the occlusal relationship to be checked is acquired; the working arch and the opposing dental arch of the three-dimensional occlusion model to display the occlusal relationship to be checked are adjusted based on the relative position matrix of the opposing dental arch and the relative position matrix of the occlusal piece, thereby meeting user requirements.

Thus, comprehensive and accurate occlusal position relationships are provided, which can, for example, help dentists better evaluate a patient's occlusal relationship, adjust the fit of restorations, and support the implementation of personalized treatment. This helps improve the accuracy and personalization of dental diagnosis and treatment.

Fig. 10 is a schematic structural diagram of a scanning processing apparatus according to an embodiment of the present invention. The apparatus may be implemented by software and/or hardware, and may generally be integrated in an electronic device. As shown in Fig. 10, the apparatus includes:
a first response acquisition module 601, configured to acquire, in response to an occlusal relationship addition request, a target dental arch and an initial occlusal relationship corresponding to the target dental arch;
a scanning module 602, configured to scan the target dental arch to obtain an occlusal piece model,
wherein the target dental arch is in a target occlusal state;
a calculation and storage module 603, configured to perform calculation based on the initial occlusal relationship and the occlusal piece model to obtain a target occlusal relationship and store the target occlusal relationship.

Optionally, the scanning processing apparatus further includes:
a first acquisition module, configured to acquire scanning request information;
a determination module configured to determine a recommended occlusal state based on the scanning request information, and display the recommended occlusal state; and
the scanning module 602 is further configured to scan the target dental arch to obtain the occlusal piece model, wherein the target dental arch is in the recommended occlusal state.

Optionally, the scanning module 602 is specifically configured to:
scan a left occlusal position of the target dental arch to obtain first occlusal piece data; and/or,
scan a right occlusal position of the target dental arch to obtain second occlusal piece data; and/or,
scan a middle occlusal position of the target dental arch to obtain third occlusal piece data; and
obtain the occlusal piece model based on at least two pieces of the first occlusal piece data, the second occlusal piece data and the third occlusal piece data.

Optionally, the calculation and storage module 603 is specifically configured to:
determine an initial relative position matrix of the target dental arch based on the initial occlusal relationship;
perform feature registration on the target dental arch and the occlusal piece model based on the initial relative position matrix, and align the target dental arch to a coordinate system of the occlusal piece model;
use the occlusal piece model as a biting matching constraint of the target dental arch, and perform constraint processing on the target dental arch and the occlusal piece model by means of a preset constraint algorithm, so as to obtain a first position transformation matrix corresponding to the target dental arch and a second position transformation matrix corresponding to the occlusal piece model, and store same as the target occlusal relationship.

Optionally, the occlusal piece model includes at least two pieces of occlusion data. The apparatus further includes:
a second acquisition module, configured to acquire a current position transformation matrix of a current piece of occlusion data and a previous position transformation matrix of a previous piece of occlusion data;
a third acquisition module, configured to acquire a target position transformation matrix of the target dental arch after constraint processing;
a first calculation module, configured to perform calculation based on the current position transformation matrix, the previous position transformation matrix and the target position transformation matrix to obtain an updated position transformation matrix of the previous piece of occlusion data.

Optionally, the apparatus further includes:
an orientation correction module, configured to perform orientation correction on the target dental arch to obtain an incremental relative position matrix;
a first processing module, configured to multiply the first position transformation matrix by the incremental relative position matrix to obtain a first target position transformation matrix corresponding to the target dental arch;
a second processing module, configured to multiply the second position transformation matrix by the incremental relative position matrix to obtain a second target position transformation matrix corresponding to the occlusal piece model;
a third processing module, configured to use the first target position transformation matrix and the second target position transformation matrix as the target occlusal relationship.

Optionally, the apparatus further includes:
a second response acquisition module, configured to an occlusal relationship identifier in response to an occlusal relationship checking request;
a fourth acquisition module, configured to acquire the target dental arch and a current occlusal relationship corresponding to the target dental arch based on the occlusal relationship identifier;
a first display module, configured to control the display of the target dental arch based on a relative position matrix corresponding to the current occlusal relationship.

Optionally, the scanning processing apparatus further includes:
a detection and acquisition module, configured to detect position update information of the target dental arch, and acquire an updated relative position matrix;
a fifth acquisition module, configured to acquire, on the basis of the initial occlusal relationship, a maxilla relative position matrix and a mandible relative position matrix of the target dental arch;
a second calculation module, configured to perform calculation based on the maxilla relative position matrix and the updated relative position matrix to obtain an updated maxilla relative position matrix of the target dental arch;
a third calculation module, configured to perform calculation based on the mandible relative position matrix and the updated relative position matrix to obtain an updated mandible relative position matrix of the target dental arch; and
a second display module, configured to control the display of the target dental arch based on the updated maxilla relative position matrix and the updated mandible relative position matrix.

Optionally, the apparatus further includes:
a response output module configured to output, in response to an occlusal relationship output request, each occlusal relationship and an opposing dental arch corresponding to each occlusal relationship,
wherein when the opposing dental arch is a mandible, a relative position matrix of the opposing dental arch corresponding to each of the occlusal relationships is a relative position inverse matrix of a maxilla multiplied by a relative position of a mandible, and a relative position matrix of the occlusal piece corresponding to each of the occlusal relationships is the relative position inverse matrix of the maxilla;
when the opposing dental arch is a maxilla, a relative position matrix of the opposing dental arch corresponding to each of the occlusal relationships is a relative position inverse matrix of a mandible multiplied by a relative position of a maxilla, and a relative position matrix of the occlusal piece corresponding to each of the occlusal relationships is the relative position inverse matrix of the mandible.

Optionally, the apparatus further includes:
a third response acquisition module configured to acquire, in response to an occlusal relationship checking request, a working arch of a three-dimensional occlusion model corresponding to the occlusal relationship to be checked;
and a third display module configured to adjust, based on the relative position matrix of the opposing dental arch and the relative position matrix of the occlusal piece, the working arch and the opposing dental arch of the three-dimensional occlusion model to display the occlusal relationship to be checked.

The apparatus for scanning processing provided by the embodiments of the present invention can perform the method for scanning processing provided in any embodiment of the present invention, and has corresponding functional modules and beneficial effects for performing the method.

An embodiment of the present invention further provides a computer program product, including a computer program/instruction. When the computer program/instruction is executed by a processor, the method for scanning processing provided in any embodiment of the present invention is implemented.

Fig. 11 is a schematic structural diagram of an electronic device according to an embodiment of the present invention. Referring now specifically to Fig. 11 which shows a block diagram of an electronic device 700 suitable for use in implementing embodiments of the present invention. The electronic device 700 in the present disclosed embodiment may include, but is not limited to, a mobile terminal such as a mobile phone, a notebook computer, a digital broadcast receiver, a personal digital assistant (PDA), a tablet computer (PAD), a portable media player (PMP), and a vehicle-mounted terminal (such as a vehicle-mounted navigation terminal), and a stationary terminal such as a digital television (TV) and a desktop computer. The electronic device shown in Fig. 11 is merely an example, and should not impose any limitation on the function and scope of use of this embodiment of the present invention.

As shown in Fig. 11, electronic device 700 may include a processing apparatus 701 (such as a central processing unit or a graphics processing unit). The processing apparatus 701 may perform various appropriate actions and processing on the basis of a program stored in a read-only memory (ROM) 702 or a program loaded from a storage apparatus 708 into a random access memory (RAM) 703. The RAM 703 further stores various programs and data required for the operation of the electronic device 700. The processing apparatus 701, the ROM 702, and the RAM 703 are connected to each other by means of a bus 704. An input/output (I/O) interface 705 is also connected to the bus 704.

In general, the following apparatus may be connected to the I/O interface 705: an input apparatus 706, including, for example, a touchscreen, a touchpad, a keyboard, a mouse, a camera, a microphone, an accelerometer, and a gyroscope; an output apparatus 707, including, for example, a liquid crystal display (LCD), a speaker, and a vibrator; a storage apparatus 708, including, for example, a magnetic tape and a hard disk; and a communication apparatus 709. The communication apparatus 709 may allow the electronic device 700 to perform wireless or wired communication with another device to exchange data. Although Fig. 11 shows the electronic device 700 having various apparatuses, it should be understood that not all the apparatuses shown here need to be implemented or provided. Alternatively, more or fewer apparatuses may be implemented or provided.

In particular, according to the embodiments of the present invention, the processes described above with reference to the flowcharts can be implemented as computer software programs. For example, an embodiment of the present invention includes a computer program product. The computer program product includes a computer program carried on a non-transitory computer-readable medium. The computer program includes program code for performing the method shown in the flowchart. In such an embodiment, the computer program may be downloaded from a network through the communication apparatus 709 and installed,
or may be installed from the storage apparatus 708, or may be installed from the ROM 702. When the computer program is executed by the processing apparatus 701, the above functions defined in the scanning processing methods of the embodiments of the present invention are performed.

It should be noted that the computer readable medium in the present invention may be a computer readable signal medium or a computer readable storage medium, or any combination thereof. The readable storage medium can be, for example, but is not limited to, an electronic, magnetic, optical, electromagnetic, infrared, or semiconductor system, apparatus, or device, or any combination thereof. More specific examples of the computer readable storage medium may include, but are not limited to, electrical connections with one or more wires, a portable computer disk, a hard disk, a random access memory (RAM), a read-only memory (ROM), an erasable Programmable read-only memory (EPROM or flash memory), an optical fiber, a portable compact disk read-only memory (CD-ROM), an optical storage device, a magnetic storage device, or any suitable combination of the above. In the present invention, the computer readable storage medium may be any tangible medium that can contain or store programs for use by or in combination with an instruction execution system, apparatus, or device. In the present invention, the computer-readable signal medium can include a data signal in baseband or propagated as part of a carrier wave, which carries readable program codes. Such a propagated data signal can take many forms, including but not limited to electromagnetic signals, optical signals, or any suitable combination of the above. The readable signal medium can also be any readable medium other than a readable storage medium, and the readable signal medium can send, propagate, or transmit a program for use by or in combination with an instruction execution system, apparatus, or device. The program codes contained on the readable medium can be transmitted through any appropriate medium, including but not limited to wired, optical fiber cable, RF, etc., or any suitable combination of the above.

In some embodiments, the client, server may communicate using any currently known or future developed network protocol such as HTTP (Hyper Text Transfer Protocol), and may be interconnected with any form or medium of digital data communication (e.g., a communication network). Examples of communication networks include local area networks ("LAN"), wide area networks ("WAN"), the Internet (e.g., the Internet), and end-to-end networks (e.g., ad hoc end-to-end networks), as well as any currently known or future-developed networks.

The computer-readable medium may be included in the electronic device, and may also exist alone without being assembled into the electronic device.

The computer-readable medium carries one or more programs, and when the one or more programs are executed by the electronic device, the electronic device is caused to: in response to an occlusal relationship addition request, acquire a target dental arch and an initial occlusal relationship corresponding to the target dental arch; scan the target dental arch in a target occlusion state to obtain an occlusal piece model; and perform calculation based on the initial occlusal relationship and the occlusal piece model to obtain a target occlusal relationship and store same.

Computer program code for performing the operations in the present invention may be written in one or more programming languages or a combination thereof, where the above-mentioned programming languages include an object-oriented programming language, such as Java, Smalltalk, and C++, and further include conventional procedural programming languages, such as "C" language or similar programming languages. The program code may be executed entirely on a user computer, partly on the user computer, as a stand-alone software package, partly on the user computer and partly on a remote computer, or entirely on the remote computer or the server. In the case of involving the remote computer, the remote computer may be connected to the user computer via any type of network, including a local area network (LAN) or a wide area network (WAN), or may be connected to an external computer (e.g., utilizing an Internet service provider for Internet connectivity).

The flowcharts and block diagrams in the drawings show possible architectures, functions, and operations of the system, method, and computer program product according to the embodiments of the present invention. In this regard, each block in the flowcharts or block diagrams may represent a module, a program segment, or part of codes containing one or more executable instructions for implementing specified logical functions. It is also to be noted that in some alternative implementations, the functions marked in the blocks may occur in an order different from those marked in the drawings. For example, two successive blocks may, in fact, be executed basically in parallel or in a reverse order, which depends on the functions involved. It is also to be noted that each block in the block diagrams and/or flowcharts and a combination of blocks in the block diagrams and/or flowcharts may be implemented by a special-purpose hardware-based system executing a specified function or operation or may be implemented by a combination of special-purpose hardware and computer instructions.

The involved units described in the embodiments of the present invention may be implemented through software or hardware. The name of the unit does not constitute a limitation on the unit itself in some cases.

Herein, the functions described above may be at least partially executed by one or more hardware logic components. For example, without limitation, exemplary hardware logic components that can be used include: a field-programmable gate array (FPGA), an application-specific integrated circuit (ASIC), an application-specific standard product (ASSP), a system on chip (SOC), a complex programmable logic device (CPLD), etc.

In the context of the present invention, a machine-readable medium may be tangible media that may contain or store programs for use by an instruction execution system, apparatus or device, or for use in connection with the instruction execution system, apparatus or device. The machine-readable medium may be a machine-readable signal medium or a machine-readable storage medium. The machine-readable medium may include, but is not limited to, an electronic, magnetic, optical, electromagnetic, infrared or semiconductor system, apparatus or device, or any suitable combinations of the described content. More specific examples of machine-readable storage media include electrical connection based on one or more wires, a portable computer disk, a hard disk, a random access memory (RAM), a read-only memory (ROM), an erasable programmable read-only memory (EPROM or flash memory), an optical fiber, a portable compact disc read-only memory (CD-ROM), an optical storage device, a magnetic storage device, or any suitable combinations of the described content.

According to one or more embodiments of the present invention, the present invention provides an electronic device, including:
a processor; and
a memory configured to store an executable instruction executable by the processor;
wherein the processor is configured to read the executable instructions from the memory and execute the instruction to implement the method for scanning processing according to any one of the embodiments of the present invention.

According to one or more embodiments of the present invention, the present invention provides a computer-readable storage medium, which stores a computer program, wherein the computer program is configured to execute the method for scanning processing according to any one of the embodiments of the present invention.

The above description is merely an illustration of the preferred embodiments and the technical principles of the present invention.

Those skilled in the art should know that the scope of present invention involved in the present invention is not limited to the technical solutions formed by specifically combining the technical features and should also cover other technical solutions formed by freely combining the technical features or equivalent features thereof without departing from the concept of the invention. for example, technical solutions formed by mutually replacing the features and (but not limited to) the technical features with similar functions disclosed in the invention.

Further, while operations are depicted in a particular order, this should not be understood to require that these operations be performed in the particular order shown or in sequential order. In certain circumstances, multitasking and parallel processing may be advantageous. Likewise, while several specific implementation details are included in the discussion above, these should not be construed as limiting the scope of the present invention. Certain features described in the context of separate embodiments may also be implemented in combination in a single embodiment. Conversely, the various features described in the context of a single embodiment may also be implemented in multiple embodiments either individually or in any suitable sub-combination.

Although the present subject matter has been described in language specific to structural features and/or methodological acts, it is to be understood that the subject matter defined in the appended claims is not necessarily limited to the specific features or acts described above. Rather, the specific features and acts described above are merely exemplary forms of implementing the claims.

### Industrial Applicability

The solution provided in the embodiments of the present invention can be applied to the technical field of scanning processing. In the embodiments of the present invention, by means of the method of acquiring, in response to an occlusal relationship addition request, a target dental arch and an initial occlusal relationship corresponding to the target dental arch; scanning the target dental arch to obtain an occlusal piece model, the target dental arch being in a target occlusal state; performing calculation based on the initial occlusal relationship and the occlusal piece model to obtain a target occlusal relationship and store same, a user can automatically add multiple groups of occlusal positions for scanning, so that data of the multiple occlusal positions can be acquired, and the technical effect of more comprehensive and accurate occlusal analysis can be provided, thereby better learning about the occlusion condition of a dental arch on the basis of the multiple groups of occlusal relationships, so as to improve the processing quality of subsequent dental restorations, dental implantation, etc.

## Claims

1. A method for scanning processing, comprising:
in response to an occlusal relationship addition request, acquiring a target dental arch and an initial occlusal relationship corresponding to the target dental arch;
scanning the target dental arch to obtain an occlusal piece model, wherein the target dental arch is in a target occlusal state; and
performing calculation based on the initial occlusal relationship and the occlusal piece model to obtain a target occlusal relationship and storing the target occlusal relationship.

2. The method for scanning processing according to claim 1, further comprising:
acquiring scanning request information;
determining a recommended occlusal state based on the scanning request information, and displaying the recommended occlusal state; and
scanning the target dental arch to obtain the occlusal piece model, wherein the target dental arch is in the recommended occlusal state.

3. The method for scanning processing according to claim 1, wherein scanning the target dental arch to obtain an occlusal piece model comprises:
scanning a left occlusal position of the target dental arch to obtain first occlusal piece data; and/or,
scanning a right occlusal position of the target dental arch to obtain second occlusal piece data; and/or,
scanning a middle occlusal position of the target dental arch to obtain third occlusal piece data; and
obtaining the occlusal piece model based on at least two pieces of the first occlusal piece data, the second occlusal piece data and the third occlusal piece data.

4. The method for scanning processing according to claim 1, wherein performing calculation based on the initial occlusal relationship and the occlusal piece model to obtain a target occlusal relationship comprises:
determining an initial relative position matrix of the target dental arch based on the initial occlusal relationship;
performing feature registration on the target dental arch and the occlusal piece model based on the initial relative position matrix, and aligning the target dental arch to a coordinate system of the occlusal piece model; and
using the occlusal piece model as an occlusion matching constraint of the target dental arch, and performing constraint processing on the target dental arch and the occlusal piece model by means of a preset constraint algorithm, so as to obtain a first position transformation matrix corresponding to the target dental arch and a second position transformation matrix corresponding to the occlusal piece model, which are taken as the target occlusal relationship.

5. The method for scanning processing according to claim 4, wherein the occlusal piece model comprises at least two pieces of occlusion data; the method further comprises:
acquiring a current position transformation matrix of a current piece of occlusion data and a previous position transformation matrix of a previous piece of occlusion data;
acquiring a target position transformation matrix of the target dental arch after constraint processing; and
performing calculation based on the current position transformation matrix, the previous position transformation matrix and the target position transformation matrix to obtain an updated position transformation matrix of the previous piece of occlusion data.

6. The method for scanning processing according to claim 5, further comprising:
performing orientation correction on the target dental arch to obtain an incremental relative position matrix;
multiplying the first position transformation matrix by the incremental relative position matrix to obtain a first target position transformation matrix corresponding to the target dental arch;
multiplying the second position transformation matrix by the incremental relative position matrix to obtain a second target position transformation matrix corresponding to the occlusal piece model; and
using the first target position transformation matrix and the second target position transformation matrix as the target occlusal relationship.

7. The method for scanning processing according to claim 1, further comprising:
acquiring an occlusal relationship identifier in response to an occlusal relationship checking request;
acquiring the target dental arch and a current occlusal relationship corresponding to the target dental arch based on the occlusal relationship identifier; and
controlling display of the target dental arch based on a relative position matrix corresponding to the current occlusal relationship.

8. The method for scanning processing according to claim 1, further comprising:
detecting position update information of the target dental arch, and acquiring an updated relative position matrix;
acquiring, on the basis of the initial occlusal relationship, a maxilla relative position matrix and a mandible relative position matrix of the target dental arch;
performing calculation based on the maxilla relative position matrix and the updated relative position matrix to obtain an updated maxilla relative position matrix of the target dental arch;
performing calculation based on the mandible relative position matrix and the updated relative position matrix to obtain an updated mandible relative position matrix of the target dental arch; and
controlling display of the target dental arch based on the updated maxilla relative position matrix and the updated mandible relative position matrix.

9. The method for scanning processing according to claim 1, further comprising:
outputting, in response to an occlusal relationship output request, each occlusal relationship and an opposing dental arch corresponding to each occlusal relationship,
wherein when the opposing dental arch is a mandible, a relative position matrix of the opposing dental arch corresponding to each of the occlusal relationships is a relative position inverse matrix of a maxilla multiplied by a relative position of a mandible, and a relative position matrix of the occlusal piece corresponding to each of the occlusal relationships is the relative position inverse matrix of the maxilla; and
when the opposing dental arch is a maxilla, a relative position matrix of the opposing dental arch corresponding to each of the occlusal relationships is a relative position inverse matrix of a mandible multiplied by a relative position of a maxilla, and a relative position matrix of the occlusal piece corresponding to each of the occlusal relationships is the relative position inverse matrix of the mandible.

10. The method for scanning processing according to claim 9, further comprising:
acquiring, in response to an occlusal relationship checking request, a working arch of a three-dimensional occlusion model corresponding to an occlusal relationship to be checked; and
adjusting, based on the relative position matrix of the opposing dental arch and the relative position matrix of the occlusal piece, the working arch and the opposing dental arch of the three-dimensional occlusion model to display the occlusal relationship to be checked.

11. An apparatus for scanning processing, comprising:
a first response acquisition module, configured to acquire, in response to an occlusal relationship addition request, a target dental arch and an initial occlusal relationship corresponding to the target dental arch;
a scanning module, configured to scan the target dental arch to obtain an occlusal piece model, wherein the target dental arch is in a target occlusal state; and
a calculation and storage module, configured to perform calculation based on the initial occlusal relationship and the occlusal piece model to obtain a target occlusal relationship and store the target occlusal relationship.

12. An electronic device, comprising:
a processor; and
a memory configured to store an executable instruction executable by the processor;
wherein the processor is configured to read the executable instruction from the memory and execute the instruction to implement the method for scanning processing according to any one of claims 1-10.

13. A computer readable storage medium, storing a computer program which is configured to execute the method for scanning processing according to any one of claims 1-10.
